(19) Europäisches Patentamt
European Patent Office
Office européen des brevets

(11) Publication number: **0 095 302 B1**

(12) **EUROPEAN PATENT SPECIFICATION**

(45) Date of publication of patent specification: **26.02.86**

(51) Int. Cl.$^4$: **G 01 N 33/72,** C 12 Q 1/28

(21) Application number: **83302770.9**

(22) Date of filing: **17.05.83**

# (54) Improved specimen slide for occult blood testing.

(30) Priority: **28.05.82 US 382908**

(43) Date of publication of application: **30.11.83 Bulletin 83/48**

(45) Publication of the grant of the patent: **26.02.86 Bulletin 86/09**

(84) Designated Contracting States: **AT BE CH DE FR GB IT LI LU NL SE**

(56) References cited:
AT-B- 358 743
AT-B- 360 176
DE-A-2 716 060
US-A-3 996 006
US-A-4 175 923

(73) Proprietor: **SMITHKLINE DIAGNOSTICS, INC.**
**485 Potrero Avenue**
**Sunnyvale California 94086 (US)**

(72) Inventor: **Baker, Josefina Tecson**
**21124 Lauretta Drive**
**Cupertino California 95014 (US)**
Inventor: **Lawrence, Paul Joseph**
**2082 Abbey Lane**
**Campbell California 95008 (US)**
Inventor: **Townsley, Charles William**
**781 Foxridge Place**
**San Jose California 95133 (US)**

(74) Representative: **Waters, David Martin, Dr.**
**Smith Kline & French Laboratories Ltd. Patent Department Mundells**
**Welwyn Garden City Hertfordshire AL7 1EY (GB)**

**EP 0 095 302 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Specimen test slides and procedures for detecting occult blood in fecal matter are well known. For example, U.S. Patent No. 3,996,006 discloses slides having a substrate consisting of a specimen receiving sheet between a front panel and a rear panel with one or more openings in the front panel and an opening in the rear panel and pivotal covers to cover these openings. Typically, in the case for a test for occult blood in feces, the specimen receiving sheet is absorbent paper impregnated or printed with guaiac and a developing solution such as a peroxide solution is applied through the opening in the rear panel. One such test slide is sold under the trademark of "Hemoccult".

Briefly, the test procedure is as follows. A sample of fecal matter is smeared onto the guaiac paper through an opening of the front panel. The panel is then covered and the flap of the rear panel is opened. A developing solution such as hydrogen peroxide is applied to the guaiac paper via the corresponding opening in the rear panel. If blood is present in the fecal matter, the guaiac reaction will color the paper blue. The blue color is due to the hemoglobin catalyzed oxidation of the guaiac.

$$\underset{\text{Colorless}}{\text{Guaiac}} \xrightarrow[\text{Hemoglobin}]{H_2O_2} \underset{\text{Blue Color}}{\text{"Oxidized" Guaiac}}$$

One of the limiting characteristics of the guaiac test is that the hemoglobin catalyzed blue color formation is pH dependent. The developing solution does not operate reproducibly below a certain pH. The pH range for the successful performance of the above known system is from about 4.0 to 7.0 with the optimum pH being from 5.5 to 6.0. Thus, if hemoglobin is applied to the guaiac treated substrate in a medium that differs significantly from the optimum pH, little or possibly no color will be detected.

Due to the above disadvantage of the present slide, it is not designed for testing the presence of occult blood in specimens having a low pH such as gastric fluid which can have a pH below 2. Detection of compounds not normally present in gastric contents, specifically the presence of occult blood, is of clinical significance. Blood in gastric contents may originate from many sources including blood swallowed subsequent to trauma outside the gastrointestinal tract and gastrointestinal bleeding. In some pathological conditions, such as carcinoma of the stomach, peptic ulcer, and gastritis, blood may be present in the stomach. Ingestion of alcohol and various drugs such as aspirin, other analgesics and corticosteroids, can cause erosions in the stomach mucosal cells and eventual gastric bleeding. Gastric erosions and subsequent bleeding have also been recorded in patients admitted to hospitals for treatment of critical injuries or burns. This type of erosion may appear within 24 hours of the original trauma and can result in acute life-threatening gastric hemorrhage. The importance of this unexpected early detection of occult blood in the gastrointestinal tract would help in diagnosing any of the above conditions at an early stage while they can be treated more effectively.

It is therefore an object of this invention to provide a specimen test slide which will not be effected by variations in sample or specimen pH and could be employed for specimens other than fecal matter.

It is also an object of this invention to provide a fast, convenient qualitative test to aid in the diagnosis of gastrointestinal conditions by the early detection of occult blood in gastric fluids.

It is a further object of this invention to provide a specimen test slide which would eliminate false-positive reactions due to the presence of peroxidases, iron, copper or chemicals such as cimetidine.

Previous attempts have been made to stabilize the guaiac impregnated paper or test strip against pH variations and interfering substances such as peroxidases. German Offenlegungschrift 2,716,060 discloses the incorporation of buffering agents or chelating agents in the guaiac paper to overcome the above stated problems. The most critical need for buffering is when the media tested differ greatly from optimum pH such as in gastric fluids, or when small amounts of catalyst such as hemoglobin are to be detected.

Unexpectedly, it was discovered that when the buffering agents were placed in the developing solution and not impregnated in the guaiac paper as disclosed in the art, the specimen test slide is not effected by variations in sample or specimen pH and false positive reactions due to the presence of peroxidases, metal ions and cimetidine were eliminated.

Several major advantages result in buffering the developing solution instead of impregnating the guaiac paper with buffers. The amount of buffering agents that could be applied to the guaiac paper is limited due to the absorbency of the paper. Therefore, when strong buffering is needed it would be more effective to buffer the developer. It would also be difficult to get an even distribution of buffers on the paper. By buffering the developer, the pH is not limited to the pH of the substrate, i.e., the pH can be changed. Once the paper is impregnated the pH is well defined and limited. A heavy salt concentration on the paper would also affect other reagents and change the physical properties of the paper itself. By buffering the developer solution there would be no change in the manufacturing procedure of the specimen test slide. This approach is very cost effective.

Most advantageous results were obtained with the combined use of acid treated guaiac paper and a buffered developer. It was discovered that acid treatment of the guaiac paper eliminated false positive tests with cimetidine and decreased interference from peroxidases. Further, unexpectedly, the acid in the guaiac

paper prevents the guaiac from turning blue during storage when atmospheric oxidation may take place. The buffered developer is needed to permit hemoglobin detection under the acidic conditions present in the guaiac. This is illustrated by the results of the following experiments.

Samples of synthetic gastric fluid containing 0.05 mg. of hemoglobin and 30 mg. of cimetidine per ml. were prepared. 25 µl samples were applied to "Hemoccult" slides and developed with buffered and unbuffered developers. The "Hemoccult" slides were then acidified by acid treatment of the guaiac paper prior to application and development of the samples. Tables 1 and 2 below demonstrate the results of these tests.

TABLE 1
Buffered *vs.* Unbuffered Developer with Regular "Hemoccult" Slides
Readings at 30 Seconds

| Sample | HEMOGLOBIN | | CIMETIDINE | |
|---|---|---|---|---|
| pH | Buffered Dev. | Unbuffered Dev. | Buffered Dev. | Unbuffered Dev. |
| 1.32 | 3 | 1 | 0 | 1 |
| 2.20 | 3 | 3 | 0 | 2 |
| 3.60 | 4 | 4 | 0 | 2 |
| 4.32 | 4 | 4 | 0 | 2 |
| 5.60 | 4 | 3 | 0 | 4 |
| 6.37 | 4 | 3 | 0 | 4 |

TABLE 2
Acidified *vs.* Unacidified Slides with
Buffered Developer

| Slides | HEMOGLOBIN | | | | | | CIMETIDINE | | | | | |
|---|---|---|---|---|---|---|---|---|---|---|---|---|
| | Acid | | | No Acid | | | Acid | | | No Acid | | |
| Reading Time (Min.) | 0.5 | 2 | 5 | 0.5 | 2 | 5 | 0.5 | 2 | 5 | 0.5 | 2 | 5 |
| Sample pH 1.32 | 2 | 5 | 5 | 3 | 5 | 5 | 0 | 0 | 0 | 0 | 0 | 0 |
| Sample pH 5.60 | 5 | 5 | 5 | 5 | 5 | 5 | 0 | 0 | 0 | 0 | 1 | 5 |
| Sample pH 6.37 | 5 | 5 | 5 | 4 | 5 | 5 | 0 | 0 | 0 | 0 | 3 | 5 |

"Hemoccult" slides were acidified by treating the center of the guaiac paper with 25 µl. of 0.1M citric acid. The paper was allowed to dry in ambient condition before using. The buffered developer contained hydrogen peroxide in a 0.2M citric acid/citrate buffer having a pH of 5.8. The unbuffered developer was the commercial "Hemoccult" developer comprising hydrogen peroxide in ethanol and water.

The blue color intensity was graded in a range of 1 to 5 with zero being colorless. It will be noted from Table 1 that the use of buffered developer on the regular "Hemoccult" slide produces a more consistent hemoglobin positive test. The buffered developer also eliminated the false positive tests caused by cimetidine. The results of Table 2 demonstrate that the use of acidified guaiac paper coupled with buffered developer will not produce a false positive result due to cimetidine even when the test readings were

delayed and done after the prescribed 30 seconds. It will be noted that this treatment did not interfere with the test for hemoglobin.

The peroxidase catalyzed false positive reaction is also pH dependent, catalysis decreases with decreasing pH. It was discovered that acid treatment of the guaiac paper prior to sample application prevents a false-positive reaction catalyzed by acid-labile peroxidases. The treated paper, when used with a buffered developer, will produce a blue color with hemoglobin containing samples and will not interfere with the test for hemoglobin. For example, synthetic gastric fluid pH 6.5 containing purified horseradish peroxidase [2 units per ml.] when tested with "Hemoccult" slides gave false positive results. When the guaiac paper was pre-treated with acid and the above synthetic gastric fluid with peroxidase was applied and allowed to react with the acid for five minutess before developing, the blue positive color was not produced.

Acids found effective for the treatment of the guaiac paper were hydrochloric, sulfuric, citric, phosphoric and oxalic. Other strong organic and inorganic acids may be used. A preferable formula and method for the acid treatment of the guaiac paper is as follows:

Solution I

| | |
|---|---|
| Guaiac | 7.8 gms. |
| Isopropanol | 92.2 gms. |

Solution II

| | |
|---|---|
| Solution I | 800 gms. |
| Citric Acid | 768 gms. |
| Isopropanol | 432 gms. |
| Water | 300 gms. |

Solution II may be applied to the absorbent paper either by impregnation or printing.

The developer may be buffered with any well known buffering system such as, for example, citric acid/sodium citrate, citric acid/dibasic sodium phosphate, tris-maleate or EDTA.

The buffered developing solution will preferably contain from about 1% to about 5% of hydrogen peroxide. The following buffered developer which is a 0.2 M. citric acid/citrate buffer having a pH of 5.8 has been advantageously employed in this invention.

| | |
|---|---|
| Hydrogen Peroxide | 2.17 gms. |
| Ethanol | 32.87 gms. |
| Citric Acid | 0.75 gms. |
| Sodium Citrate | 4.37 gms. |
| Water | 58.80 gms. |
| Triton X—100 | 1.04 gms. |

Preferably a nonionic detergent is added to the buffered developer in order to assist in the peroxidase inhibition. Most advantageously a Triton X® detergent, polyethylene glycol p-isooctylphenyl ether, is employed.

When a buffered developer is applied to the substrate of acid treated guaiac paper a specimen test slide results which is stable and permits testing for the presence of occult blood in specimens having a low pH such as gastric fluid. Further, false positive results due to cimetidine, peroxidases and metal ions such as copper and iron are eliminated.

**Claims**

1. A specimen test slide for testing occult blood comprising an absorbent substrate treated with strong acid in the absence of buffer and containing guaiac as a test reagent.

2. The slide of Claim 1 wherein the substrate comprises a sheet of absorbent paper.

3. The slide of Claim 1 wherein the acid is citric acid.

4. A method of testing for occult blood in gastric fluid which comprises applying the gastric fluid to an

acid treated absorbent substrate containing guaiac as a reagent and applying a buffered developing solution having a pH of about 5.8 to said substrate and observing if the substrate is colored blue.

5. The method of Claim 4 wherein the developing solution contains hydrogen peroxide.

6. A test kit for determining the presence of occult blood in gastric fluid which comprises an acid treated absorbent substrate containing guaiac reagent and a receptacle containing a buffered developing solution which reacts with said reagent to color the substrate blue in presence of blood.

7. An aqueous developing solution comprising from about 1% to about 5% of hydrogen peroxide buffered by 0.2 M citric acid/sodium citrate having a pH of about 5.8.

8. The solution of claim 7 wherein a nonionic detergent is added.

**Patentansprüche**

1. Probenteststreifen zur Prüfung auf okkultes Blut, gekennzeichnet durch ein absorbierendes Substrat, das mit einer starken Säure in Abwesenheit von Puffer behandelt wurde und das Guajakharz als Testreagens enthält.

2. Streifen nach Anspruch 1, dadurch gekennzeichnet, daß das Substrat ein Blatt saugfähiges Papier enthält.

3. Streifen nach Anspruch 1, dadurch gekennzeichnet, daß die Säure Citronensäure ist.

4. Verfahren zur Bestimmung der Anwesenheit von okkultem Blut im Magensaft, dadurch gekennzeichnet, daß man den Magensaft auf ein mit Säure behandeltes absorbierendes Substrat aufbringt, das Guajakharz als Reagens enthält, eine gepufferte Entwicklerlösung mit einem pH-Wert von etwa 5,8 auf das Substrat aufbringt und beobachtet, ob das Substrat sich blau verfärbt.

5. Verfahren nach Anspruch 4, dadurch gekennzeichnet, daß die Entwicklerlösung Wasserstoffperoxid enthält.

6. Testbesteck zur Bestimmung der Anwesenheit von okkultem Blut im Magensaft, gekennzeichnet durch ein mit Säure behandeltes absorbierendes Substrat, das Guajakharz als Reagens enthält, und ein eine gepufferte Entwicklerlösung, die in Anwesenheit von Blut mit dem Reagens reagiert und das Substrat blau färbt, enthaltendes Gefäß.

7. Wäßrige Entwicklerlösung, dadurch gekennzeichnet, daß sie etwa 1 bis etwa 5% Wasserstoffperoxid, gepuffert mit 0,2 m Citronensäure/Natriumcitrat mit einem pH-Wert von etwa 5,8, enthält.

8. Lösung nach Anspruch 7, dadurch gekennzeichnet, daß sie zusätzlich ein nicht-ionisches Netzmittel enthält.

**Revendications**

1. Un porte-objet d'éprouvettes pour faire des tests de sang invisible à l'oeil nu, comprenant un substrat absorbant traité avec un acide fort en l'absence de tampon et qui contient du guaiac en tant que réactif de test.

2. Le porte-objet de la revendication 1 dans lequel le substrat comprend une feuille de papier absorbant.

3. Le porte-objet de la revendication 1 dans lequel l'acide est de l'acide citrique.

4. Une méthode pour faire des tests sur le sang invisible à l'oeil nu dans la suc gastrique, qui comprend l'application de suc gastrique à un substrat absorbant traité à l'acide et contenant du guaiac en tant que réactif et l'application d'une solution tamponnée de révélateur, ayant un pH de 5,8 environ, sur le dit substrat et de voir si le substrat se colore au bleu.

5. La méthode de la revendication 4 dans laquelle la solution de révélateur contient de l'eua oxygénée.

6. Un nécessaire pour faire des tests en vue de déterminer la présence de sang invisible à l'oeil nu dans le suc gastrique, et qui comprend un substrat absorbant traité à l'acide contenant un réactif de guaiac et un récipient contenant une solution de révélateur tamponnée qui réagit avec ledit réactif pour colorer le substrat en bleu en présence de sang.

7. Une solution de révélateur aqueuse, comprenant entre 1% et 5% environ de peroxyde pour 0,2 M d'acide citrique/citrate de sodium, ayant un pH de 5,8 environ.

8. La solution de la revendication 7 dans laquelle on ajoute un détergent non ionique.